(19)

Europäisches
Patentamt
European
Patent Office
Office européen
des brevets

(11)     **EP 2 787 070 A2**

(12)     **EUROPEAN PATENT APPLICATION**

(43) Date of publication:
**08.10.2014   Bulletin 2014/41**

(51) Int Cl.:
**C12N 1/06** *(2006.01)*          **C12N 9/34** *(2006.01)*
**C12G 1/00** *(2006.01)*

(21) Application number: **14174911.9**

(22) Date of filing: **21.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(62) Document number(s) of the earlier application(s) in accordance with Art. 76 EPC:
**11174893.5 / 2 548 944**

(71) Applicant: **AB Enzymes GmbH**
**64293 Darmstadt (DE)**

(72) Inventors:
• **Langfelder, Kim**
**64285 Darmstadt (DE)**
• **Marschner, Volker**
**64404 Bickenbach (DE)**

• **Titze, Kornelia**
**64367 Mühltal (DE)**
• **Alapuranen, Marika**
**04300 Tuusula (FI)**
• **Vehmaanperä, Jari**
**01800 Klaukkala (FI)**

(74) Representative: **Hiebl, Inge Elisabeth**
**Kraus & Weisert**
**Patentanwälte PartGmbB**
**Thomas-Wimmer-Ring 15**
**80539 München (DE)**

Remarks:
This application was filed on 30-06-2014 as a divisional application to the application mentioned under INID code 62.

(54)     **Process of lysing yeast cell walls**

(57)     The invention relates to the use of a composition comprising at least one polypeptide having glucoamylase activity and optionally at least one polypeptide having β-1,3-endoglucanase activity in a process of lysing yeast cell walls as well as to a process for lysing yeast cell walls which comprises exposing said yeast cell walls to a composition comprising at least one polypeptide having glucoamylase activity and optionally at least one polypeptide having β-1,3-endoglucanase activity.

EP 2 787 070 A2

**Description**

The invention relates to an enzyme composition and to a process for the lysis of yeast cell walls using a glucoamylase enzyme activity or a combination of two enzyme activities, i.e., a glucoamylase activity and an β-1,3-endoglucanase activity. Specifically, the invention relates to the use of polypeptides having glucoamylase activity to lyse yeast cells and an enzyme preparation comprising at least one polypeptide having glucoamylase activity and at least one polypeptide having β-1,3-endoglucanase activity in a process of lysing yeast cell walls which comprises exposing said yeast cell walls to a composition comprising at least one polypeptide having glucoamylase activity and at least one polypeptide having β-1,3-endoglucanase activity.

Various kinds of yeasts are used and/or produced in various kinds of industries such as the biofuel industry, the food industry, the feed industry, the pharmaceutical industry etc. In technologies using yeast cells for the production of food or feed like in processes for making wine or beer or processes for producing feed yeasts or particles thereof tend to adhere to the production devices such as filters and crossflow membranes and eventually spoil and clog them. Cleaning of said clogged and spoiled devices is usually most efficiently done by lysing the yeast cells and cell debris.

In processes where the availability of the yeast cell material is of importance, such as the preparation of yeast lysates or the generation of bioactive peptides from yeast for animal and human purposes, the availability of the yeast cell material may be increased by removal of yeast cell walls and cell debris.

Various processes for lysing yeast cell walls have been used and proposed in the art. Apart from physical and chemical treatments such as treatment with heat, concentrated acids or concentrated alkaline solutions, enzymatic treatment of yeast cell walls is usually considered most advantageous.

To date, two major activities have been shown to cause yeast (*Saccharomyces cerevisiae*) lysis, ß-endo-1,3-endoglucanase (laminarinase) and protease (Scott and Scheckman, 1980; Salazar and Asenjo, 2007, Enzymatic lysis of Microbial cells. Biotech. Lett. 29: 985-994). There are also patent applications which claim methods to hydrolyse yeast and which show that the above enzymes are the most critical enzymes for yeast hydrolysis (e.g., WO 96/23579, WO2008/037777, WO 2008/110632, WO 2007/042577). These findings are confirmed by an understanding of the yeast cell-wall structure which according to the art consists mostly of mannoprotein and ß-1,3-glucan (i.e., glucose molecules linked by ß-1,3 linkages). There is also ß-1,6-glucan (i.e., glucose molecules linked by ß-1,6 linkages) and a small amount of chitin (N-acetylglucosamine linked by ß-1,4-glycosidic bonds) (Lipke and Ovalle, 1998; "Cell Wall Architecture in Yeast: New Structure and New Challenges", Table 2).

According to current literature (Lipke and Ovalle, 1998, "Cell Wall Architecture in Yeast: New Structure and New Challenges"; Cabib et al., 1991, "Carbohydrates as structural constituents of yeast cell wall and septum", Pure & Appl. Chem., 63: 483-489; Manners et al., 1973, "The Structure of a ß-1,3-glucan from yeast cell walls", Biochem. J. 135:19-30) the ß-1,3-glucans form the main structural component of the cell wall in the form of a fibrous network of α-helical modules. The ß-1,6-glucan is a highly branched polysaccharide that links the components of each module together. The chitin is attached to the ß-1,3-glucan modules and probably chitin from different modules anneals to form crystalline domains. The mannoproteins in the yeast cell wall are highly glycosylated. Many glycans on the outer side of the yeast cell wall are chains of 50 to 200 α-1,6-mannose units and may have additional α-1,2 and α-1,3-sidechains. The mannoprotein chains are not crucial for cell-wall integrity.

Consequently, the enzymes described in the prior art that are considered effective to lyse yeast cell walls are endo-ß-1,3-glucanases (laminarinases), since approximately 50% of the cell wall consists of ß-1,3-glucan, ß-1,6-endoglucanases, which cross-link the modules of ß-1,3-glucan, and proteases, which degrade the mannoprotein.

The enzymatic methods of the prior art for lysing yeast cell walls have certain disadvantages or are not considered fully satisfactory. Current enzymatic methods are slow and do not lyse sufficiently well or completely so that the yield of lysed yeast-cell walls is low or the membrane or filter or filter aid is not cleaned and regenerated to the original level. In specific applications side-activities may be problematic especially with regard to generating undesired flavours.

It is, therefore, an object of the present invention to provide a process for lysing yeast cell walls that avoids the various disadvantages of the prior art. The process of lysing yeast cell walls should be highly effective, applicable to various purposes, where yeast cell-wall lysis is needed, should be effective at relatively low reaction temperatures and should be applicable to cell walls from a wide variety of yeasts. Preferably no or no cost-intensive pretreatment of the yeast cell walls to be lysed should be necessary. Moreover, the process should not lead to toxic or environmentally disadvantageous products. Moreover, the process should be applicable to various industrially relevant processes. In particular, the process for lysing yeast cell walls should be particularly suitable in a process for cleaning filter membranes or filter cartridges or filter aids or in a process for manufacturing wine.

The object of the invention is solved by the use of a preparation comprising at least one polypeptide having glucoamylase activity optionally together with at least one polypeptide having β-1,3-endoglucanase activity in a process of lysing yeast cell walls as well as by a process of lysing yeast cell walls which comprises exposing said yeast cell walls to a composition comprising at least one polypeptide having glucoamylase activity and optionally at least one polypeptide having β-1,3-endoglucanase activity.

It has surprisingly been found that an enzyme having glucoamylase activity was effective at lysing yeast cell walls and that a preparation further comprising an enzyme having β-1,3-endoglucanase activity leads to a much higher degree of yeast cell wall lysis than the use of each of said enzymes alone. Said finding must be deemed surprising since in the art it had been known that laminarinases (ß-1,3-endoglucanases) alone or in combination with proteases have a lytic effect on yeast cells. It has surprisingly been found that at the same endoglucanase dosage the rate of lysis of yeast cell walls was much faster and much more complete when glucoamylase was added than when ß-1,3-endoglucanase was used alone. Said effect was not to be expected and surprising because glucoamylase is an enzyme which hydrolyses only the $\alpha$-1,4 and $\alpha$-1,6-linked glucose bonds from starch and this type of bond has not been described in the prior art in relation to yeast cell walls.

On the basis of the above finding, various industrial processes, wherein the lysis of yeast cell walls is of importance, may be improved. Such processes are for example the membrane cleaning/the filter cartridge cleaning in the brewing industry, wherein an improved membrane cleaning is to be achieved. A further application is the *sur lie* process which makes use of yeast lysis/autolysis in wine and champagne making which leads to an increased aroma development in wine. Moreover, the process/use of the invention may be used to improve processes for creating yeast lysates.

According to the present invention, any polypeptide having glucoamylase activity can be used. The polypeptide having glucoamylase activity may be a fungal glucoamylase, a bacterial glucoamylase, a yeast glucoamylase, a plant glucoamylase, a recombinant glucoamylase or an active fragment thereof. Preferably, the enzyme to be used in the processes of the invention is a glucoamylase (E.C.3.2.1.3) derived from a microorganism or a plant. Preferred are glucoamylases of fungal or bacterial origin selected from the group consisting of *Aspergillus* glucoamylases, in particular *A. niger* G1 or G2 glucoamylase (Boel et al. (1984), EMBO J. 3 (5), p. 1097-1102), or variants thereof, such as disclosed in WO92100381 and WO00/04136; the *A. awamori* glucoamylase (WO084/02921), *A. oryzae* (Agric. Biol. Chem. (1991), 55 (4), p. 941-949), or variants or fragments thereof, *Trichoderma* glucoamylases, in particular *Trichoderma reesei* glucoamylases such as those disclosed in US 7,413,879, and variants or fragments thereof, *Hormoconis glucoamylases,* in particular *Hormoconis resinae* glucoamylase as described in US 5,665,585, and variants or fragments thereof.

Other contemplated *Aspergillus* glucoamylase variants include variants to enhance the thermal stability: G137A and G139A (Chen et al. (1996), Prot. Engng. 9, 499-505); D257E and D293E/Q (Chen et al. (1995), Prot. Engng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Engng. 10, 1199-1204). Other contemplated glucoamylases include *Talaromyces* glucoamylases, in particular derived from *Talaromyces emersonii* (WO99/28448), *Talaromyces leycettanus* (U.S. Pat. No. Re. 32,153), *Talaromyces duponti, Talaromyces thermophilus* (U.S. Pat. No. 4,587,215). Bacterial glucoamylases contemplated include glucoamylases from the genus *Clostridium,* in particular *C. thermoamylolyticum* (EP 0 135 138), and *C. thermohydrosulfuricum* (WO86/01831). Preferred glucoamylases include the glucoamylases derived from *Aspergillus oryzae,* such as a glucoamylase having at least 90%, at least 92%, at least 95%, at least 96%, at least 97%, at least 98%, or particularly at least 99% identity to the amino acid sequence shown in SEQ ID NO:2 in WO00/04136. Also contemplated are the commercial products such as Gammadex CAL (AB Enzymes, Glucoamylase preparation from *Aspergillus niger*) but corresponding products from other companies may be used as well. Glucoamylases may be added in effective amounts well known to the person skilled in the art.

According to the present invention any polypeptide having β-1,3-endoglucanase activity may be used. The polypeptide having β-1,3-endoglucanase (synonymous: endo-β-1,3-glucanase) activity may be a fungal endoglucanase, a bacterial endoglucanase, a yeast endoglucanase, a plant, a recombinant endoglucanase or an active fragment thereof.

Preferably, the polypeptide having endoglucanase activity is a ß-1,3-endoglucanase also called laminarinase (E.C.3.2.1.39 and E.C. 3.2.1.6, Enzyme Nomenclature, Academic Press, Inc, 1992) from a microorganism or plant, more preferably of fungal or bacterial origin, even more preferably from filamentous fungi, most preferably selected from the group consisting of *Aspergillus* endo-ß-1,3-glucanases, in particular *A. niger* endo-ß-1,3-glucanase (Weifen et al. (2000), Genbank Accession Number AAG01165) or variants thereof, *Trichoderma* endo-ß-1,3-glucanase, in particular *Trichoderma reesei* endo-ß-1,3-glucanases, *T. harzianum* endo-ß-1,3-glucanase, *T. viride* endo-ß-1,3-glucanase or fragments or variants thereof (Nobe et al., (2004), Biosci. Biotechnol. Biochem. 68: 2111-2119), *Penicillium* endo-ß-1,3-glucanase, in particular *Penicillium emersonii* endo-ß-1,3-glucanase and variants or fragments thereof (Murray et al. (2001) Enz. Microb. Technol. 29, 90-98) or *Oerksovia* endo-ß-1,3-glucanase, in particular *Oerksovia xanthineolytica* endo-ß-1,3-glucanase such as those disclosed in US 6,284,509, and variants or fragments thereof.

Other contemplated endo-ß-1,3-glucanase variants include polypeptides derived from *Bacillus,* in particular *Bacillus licheniformis* endo-ß-1,3-glucanase and variants or fragments thereof (Lloberas et al., 1991 Europ. J. Biochem. 197: 347-353), *B. amyloliquefaciens* endo-ß-1,3-glucanase and variants or fragments thereof (Hofemeister et al., (1986), Gene 49:177-187), *B. macerans* endo-ß-1,3-glucanase and variants or fragments thereof (Hofemeister et al., (1988) J. Basic Microbiol., 28: 1-10) or *B. subtilis* endo-ß-1,3-glucanase and variants or fragments thereof (Cantwell et al., (1983), Gene 23: 211-219) and other bacterial laminarinases such as those derived from *Thermotoga* species, such as *T.*

*neapolitana* and variants or fragments thereof (Zverlov et al., (1997) Microbiol. 143: 1701-1708). Such enzymes are commercially available or may be produced according to acknowledged processes in the art (see, for example, US 6,284,509).

In the process of the invention for lysing yeast cell walls the activity ratio of the polypeptide having endoglucanase activity and the polypeptide having glucoamylase activity is between 100.000 LAM : 1 GAU and 1:100, preferably between 10.000:1 and 1:10, more preferably between 1.000:1 and 1:1 and even more preferably between 300:1 and 10:1.

The above enzyme activities may be combined with other yeast cell-wall degrading enzyme activities such as peptidases (i.e. enzymes that are capable of cleaving peptide bounds; proteases, proteinases), beta-1,6-glucanases, chitinases, mannanases, lipases, esterases and alpha-1,3-glucanases.

Exemplary compositions for use in the process of the present invention may comprise at least one polypeptide having glucoamylase activity, at least one polypeptide having $\beta$-1,3-endoglucanase activity and one or more of the above other yeast cell-wall degrading enzyme activities. Further contemplated are compositions comprising at least one polypeptide having glucoamylase activity together with one or more of the above other yeast cell-wall degrading enzyme activities. Preferred compositions for use according to the present invention are compositions comprising a polypeptide having glucoamylase activity and a polypeptide having peptidase activity. A further preferred composition is a composition comprising a polypeptide having glucoamylase activity, a polypeptide having $\beta$-1,3-endoglucanase activity and a polypeptide having peptidase activity. In the above preferred compositions one or more polypeptide(s) having the indicated activity may be present. The preferred compositions may consist of the indicated enzyme activities. According to the present invention preferably a composition comprising at least one polypeptide having glucoamylase activity and at least one polypeptide having endoglucanase activity is used.

In the above preferred embodiment the polypeptide having glucoamylase activity and the polypeptide having peptidase activity may be combined such that the activity ratio is between 2000 $UHb_{4,4}$: 1 GAU and 1:2000, preferably between 500:1 and 1:500, even more preferably 100:1 and 1:100 and most preferably between 60:1 and 1:60.

In the above further preferred embodiment the polypeptide having glucoamylase activity and the polypeptide having ß-1,3-endoglucanase activity and the polypeptide having protease activity may be combined such that the activity ratio of the polypeptide having ß-1,3-endoglucanase activity and the polypeptide having glucoamylase activity is between 100.000 LAM : 1 GAU and 1:100, preferably between 10.000:1 and 1:10, more preferably between 1.000:1 and 1:1 and even more preferably between 300:1 and 10:1 while the ratio of the polypeptide having protease activity and the polypeptide having glucoamylase activity is between 2000 $UHb_{4,4}$: 1 GAU and 1:2000, preferably between 500:1 and 1:500, even more preferably 100:1 and 1:100 and most preferably between 60:1 and 1:60.

As a polypeptide having peptidase activity basically any polypeptide exhibiting peptidase activity and specifically any polypeptide exhibiting peptidase activity and being active at pH 3.0-11.0 may be used.

The polypeptide having peptidase activity may be a fungal peptidase, a bacterial peptidase, a yeast peptidase, a plant peptidase, a recombinant peptidase or an active fragment thereof. Preferably the peptidase is active in the pH range from pH 3.0 to 10.0, more preferably from pH 4.0 to 9.0 and most preferably from 5.0 to 8.0. Preferably the peptidase has a temperature optimum between 25°C and 85°C, more preferably between 30°C and 70°C and most preferably between 35°C and 60°C. Contemplated peptidase enzymes include polypeptides derived from plant sources, in particular from *Carica sp.,* such as *Carica papaya,* fungal sources, in particular from *Aspergillus sp.,* such as *Aspergillus niger* and *Aspergillus oryzae,* from *Trichoderma sp.,* such as *T. reesei, T. harzianum* and *T. viride,* and bacterial sources such as *Bacillus sp.,* in particular *B. subtilis, B. stearothermophilus and Streptomycetes* such as *Oerksovia sp.,* in particular *Oerksovia xanthineolytica (*Adamitsch et al., (2003) Lett. App. Microbiol. 36:227-229; Conway et al., (2001) Can. J. Microbiol. 47:18-24; Chao et al., US 4,218,418). Among the types of peptidases that can be used are serine proteases (Shimoi et al., (1992), J. Biol Chem 267: 25189-25195).

Preferably the peptidase is a cystein peptidase. More preferably the peptidase is selected from ficin, papain, or bromelain. Most preferably the peptidase is papain.

Examples for the above peptidases are well known in the art and commercially available to a person skilled in the art.

The other above enzyme activities for use according to the present invention such as ß-1,6-glucanases, chitinases, mannanases, lipases, esterases and $\alpha$-1,3-glucanases are well known in the art and easily available to a person skilled in the art.

By the term "active fragment thereof" as used in connection with an enzyme activity is meant any fragment of a polypeptide having the indicated enzyme activity but may not have the full length of the enzyme, i.e. it may be shorter than the complete enzyme but still has the indicated enzyme activity. The active fragment of an enzyme may be combined with further protein additions provided that the activity of the indicated enzyme is maintained. The term "variants" of an enzyme denotes modifications of an enzyme by addition, substitution, deletion or otherwise leading to a protein that still has the indicated enzyme activity.

The term "polypeptide having a defined enzyme activity" is to denote a polypeptide that has the indicated enzyme activity as such but may also have other enzyme activities in addition to the indicated enzyme activity.

Moreover, other ingredients may be incorporated into the composition for use according to the present invention such as inorganic salts, emulsifiers, ionic and non-ionic surfactants, stabilizers such as sorbitol, glycerol, preservatives such as sodium benzoate, alcohols, alone or in combination.

The process/use according to the present invention is suitable for lysing the cell walls of any kind of yeast, for example *Torula* species such as *T. utilis,* baker's yeast, brewer's yeast, *Saccharomyces* species such as S. *cerevisiae, Schizosaccharomyces* species such as *Schizosaccharomyces pombe, Pichia* species such as *P. pastoris, Candida* species such as C. *albicans, Hansenula* species such as *H. polymorpha* and *Kluyveromyces* species such as *K. lactis* or a mixture of any of these. The yeast cells may be grown purposely for manufacture of lysates or may be side products from other processes, such as ethanol production and others.

The process of the invention for lysing yeast cell walls using the claimed enzyme activities may be carried out as follows: a preparation of yeast cells, which optionally is pretreated chemically or physically beforehand, is incubated with a preparation containing one or more enzymes. Following this, the yeast preparation is optionally washed, to remove the enzymes and is optionally treated further to achieve additional lysis of the cells or more complete purification of the filter in question.

The process of the invention may be advantageously used within the scope of other technical processes. Examples of such processes are described in the following:

A process for cleaning of crossflow-filtration modules may be conducted as follows: in the first step the crossflow module may be treated with alkaline solutions, such as sodium hydroxide solution, having a pH range of 8-14, preferably of 9-13, more preferably of 10-12. The treatment with the alkaline solution lasts in the range of 5 minutes to 30 minutes, preferably 10-20 minutes at a temperature of 20 to 80°C, preferably 50-75°C, more preferably 60-70°C. Following this the crossflow module may be rinsed with water for 10 to 30 minutes to remove the alkaline solution before treating the cartridge with an enzyme solution according to the claimed use. The treatment with the enzyme composition will be carried out at a dosage of 200 LAM/g to 2000 LAM/g in the cleaning solution, preferably at 500 LAM/g to1500 LAM/g at a pH of 4 to 6, preferably of 4.5 to 5 and at a temperature of 30 to 60°C, preferably of 40 to 50°C for 20 to 120 minutes, preferably from 30 to 60 minutes. After enzymatic treatment the crossflow module may be washed with alkaline solutions, such as sodium hydroxide solution, having a pH range of 8-14, preferably of 9-13, more preferably of 10-12. The treatment with the alkaline solution can last in the range of 5 to 30 minutes, preferably 10-20 minutes at a temperature of 20 to 80°C, preferably 50-75°C, more preferably 60-70°C. Alternatively the crossflow module may be rinsed with acidic solutions, such as nitric acid solution, having a concentration of 0.5 to 5%, preferably 1-2%. The treatment with the acid solution can last in the range of 5 to 30 minutes, preferably 10-20 minutes at a temperature of 20 to 80°C, preferably 50-75°C, more preferably 60-70°C. After the treatment with alkaline or acidic solution the crossflow module can be rinsed with water to remove the acidic solution or the alkaline solution. In the case that no acidic or alkaline solution was used the crossflow module is washed with water to remove the enzyme solution. The washing is carried out with water for 10-30 minutes before the crossflow module can be reused.

A process for cleaning of filter aids such as co-extrudates consisting of a thermoplastic polystyrene component and a non-thermoplastic water-insoluble crosslinked polyvinylpyrrolidon, such as the commercially available product CROSSPURE® (BASF AG) may be conducted as follows: in the first step the filter aid may be treated with alkaline solutions, such as sodium hydroxide solution, having a pH range of 8-14, preferably of 9-13, more preferably of 10-12. The treatment with the alkaline solution can last in the range of 10 minutes to 180 minutes, preferably 30-120 minutes at a temperature of 20 to 80°C, preferably 50-75°C, more preferably 60-70°C. Following this the filter aid may be rinsed with water or an acidic solution, such as nitric acid solution for 10-30 minutes to remove the alkaline solution before treating the filter aid with an enzyme solution according to the claimed use. During the enzymatic cleaning of the filter aid the filter aid-slurry may be stirred to improve the efficiency of the enzymatic treatment. The treatment with the enzyme composition will be carried out at a dosage of 200 LAM/g to 2000 LAM/g in the cleaning solution, preferably at a dosage of 500 LAM/g to 1500 LAM/g at a pH of 4 to 6, preferably of 4.5 to 5 and at a temperature of 30 to 60°C, preferably of 40 to 50°C for 20 to 300 minutes, preferably 30 to 120 minutes. After enzymatic treatment the filter aid may be washed with a solution containing surfactants such as ionic and non-ionic surfactants. Such surfactants are commercially available. After treatment with enzyme solution or after treatment with surfactant solution, if this is desired, the filter aid is rinsed with hot or cold water to remove the enzyme solution and / or the surfactant solution. The washing is carried out with water for 10-30 minutes before the filter aid can be reused.

A process for cleaning of filter cartridges may be conducted as follows: in the first step the filter cartridge may be treated with alkaline solutions, such as sodium hydroxide solution, having a pH range of 8-14, preferably of 9-13, more preferably a of 10-12. The treatment with the alkaline solution can last in the range of 10 minutes to 60 minutes, preferably 20-30 minutes at a temperature of 20 to 90°C, preferably 60-90°C, more preferably 80-90°C. Following this the filter cartridge may be rinsed with water for 10-30 minutes to remove the alkaline solution before treating the filter cartridge with an enzyme solution according to the claimed use. The treatment with the enzyme composition will be carried out at a dosage of 200 LAM/g to 2000 LAM/g in the cleaning solution, preferably at 500 LAM/g to 1500 LAM/g at a pH of 4 to 6, preferably of 4.5 to 5 and at a temperature of 30 to 60°C, preferably 40 to 50°C for 20 to 120 minutes, preferably for 30 to 60 minutes. After enzymatic treatment the filter cartridge may be washed with alkaline solutions, such as sodium hydroxide solution, having a pH range of 8-14, preferably of 9-13, more preferably of 10-12. The treatment with the alkaline solution can last in the range of 5 to 30 minutes, preferably 10-20 minutes at a temperature of 20 to 80°C, preferably 50-75°C, more preferably 60-70°C. After enzymatic treatment the filter cartridge may be washed with a solution containing surfactants such as ionic and non-ionic surfactants. Such surfactants are commercially available. After the treatment with alkaline solution and optionally treatment with surfactant the filter cartridge can be rinsed with water to remove the alkaline solution. In the case that no alkaline solution was used the filter cartridge is washed with water to remove the enzyme solution. The washing is carried out with water for 10-30 minutes before the filter cartridge can be reused.

A process for accelerated yeast lysis in manufacturing sparkling wine may be conducted as follows: following the method used for methode traditionelle / method champagnoise the blended wine will be fermented a second time. For this the blended wine is placed in bottles along with yeast and a small amount of sugar. The amount of sugar added is 18 to 24 grams per liter, preferably 18 to 20 grams per liter. In addition other liquids may be added such as wine. The amount of *Saccharomyces cerevisiae* yeast added, is between 10 and 60 g/hl, preferably between 20 and 40 g/hl. At this stage, the enzyme preparation according to the claimed use is added as well. The dosage of enzyme preparation can be 500 LAM/liter to 3000 LAM/liter of wine, preferably 600 to 2500 LAM/liter. In the process the wine may be stored for 15 months to 7 years, at temperatures of 10°C to 24°C, preferably 16°C to 22°C, depending on the desired properties of the sparkling wine. Following storage the lees will be removed through riddling and disgorgement, according to the state of the art.

A process for accelerated yeast lysis in manufacturing wine may be conducted as follows: following preparation of the must this is placed in bottles or barrels along with yeast. In addition other liquids or additives may be added such as sugar or nutrients. The amount of *Saccharomyces cerevisiae* yeast added is between 10 and 60 g/hl, preferably between 20 and 40 g/hl. At this stage, the enzyme preparation claimed is added as well. The dosage of enzyme preparation is 500 LAM/liter to 3000 LAM/liter of wine, preferably 600 to 2500 LAM/liter. In the process the wine may be stored for between 1 week and 7 years, at temperatures of 10°C to 24°C, preferably 16°C to 22°C, depending on the desired properties of the sparkling wine. Following storage the lees will be removed through riddling and disgorgement, according to the state of the art.

The process of lysing yeast cell walls using an ß-1,3-endoglucanase activity and a glucoamylase activity has the following advantages: the use of glucoamylase alone or in combination with ß-1,3-endoglucanase and / or other enzymes leads to a faster and more complete lysis of the yeast cells and a more complete lysis of the yeast-cell walls compared to the state of the art, allowing for shorter processing times, more efficient lysis and more efficient cleaning. For some processes the use of the enzymes alone or in combination will lead to a reduction in processing times while producing more desirable aroma in the lysate or the wine and may also lead to better storage stability of the wine.

The present invention also relates to the following items:

1. Use of a composition comprising at least one polypeptide having glucoamylase activity optionally together with at least one polypeptide having β-1,3-endoglucanase activity in a process of lysing yeast cell walls.

2. The use of item 1, wherein the composition further comprises an enzyme selected from peptidases, mannanases, lipases, esterases, beta-1,6-glucanases, alpha-1,3-glucanases alone or in combination.

3. The use of one of items 1 or 2, wherein the composition comprises one or more polypeptide(s) having glucoamylase activity, one or more polypeptide(s) having β-1,3-endoglucanase activity and one or more polypeptide(s) having peptidase activity.

4. The use of one of items 1 to 3, wherein the polypeptide having glucoamylase activity is selected from fungal glucoamylases, bacterial glucoamylases, yeast glucoamylases, plant glucoamylases, recombinant glucoamylases or an active fragment thereof.

5. The use of one of items 1 to 4, wherein the polypeptide having β-1,3-endoglucanase activity is selected from fungal endoglucanases, bacterial endoglucanases, yeast endoglucanases, plant endoglucanases, recombinant endoglucanases or an active fragment thereof.

6. The use of one of items 1 to 5, wherein the activity ratio of the polypeptide having glucoamylase activity and the polypeptide having β-1,3-endoglucanase activity is between 1 GAU : 100.000 LAM and 100 GAU : 1 LAM.

7. A process of lysing yeast cell walls which comprises exposing said yeast cell walls to a composition comprising at least one polypeptide having glucoamylase activity and optionally at least one polypeptide having β-1,3-endoglucanase activity.

8. The process of item 7, wherein the composition further comprises an enzyme selected from peptidases, mannanases, lipases, esterases, beta-1,6-glucanases, alpha-1,3-glucanases alone or in combination.

9. The process of one of items 7 or 8, wherein the composition comprises one or more polypeptide(s) having glucoamylase activity, one or more polypeptide(s) having β-1,3-endoglucanase activity and one or more polypeptide(s) having peptidase activity.

10. The process of one of items 7 to 9, wherein the polypeptide having glucoamylase activity is selected from fungal glucoamylases, bacterial glucoamylases, yeast glucoamylases, plant glucoamylases recombinant glucoamylases or an active fragment thereof.

11. The process of one of items 7 to 10, wherein the polypeptide having β-1,3-endoglucanase activity is selected from fungal endoglucanases, bacterial endoglucanases, yeast endoglucanases, plant glucoamylases, recombinant endoglucanases or an active fragment thereof.

12. The process of one of items 7 to 11, wherein the activity ratio of the polypeptide having glucoamylase activity and the polypeptide having endoglucanase activity is between 1 GAU : 100.000 LAM and 100 GAU : 1 LAM.

13. A process for the preparation of a yeast cell lysate, which comprises the use of one of items 1 to 6 or a process of one of items 7 to 12.

14. A process for cleaning filter membranes or filter cartridges, which comprises the use of one of items 1 to 6 or a process of one of items 7 to 12.

15. A process for manufacturing a wine, which comprises the use of one of items 1 to 6 or a process of one of items 7 to 12.

The enclosed Figures serve to illustrate the subject-matter of the invention.

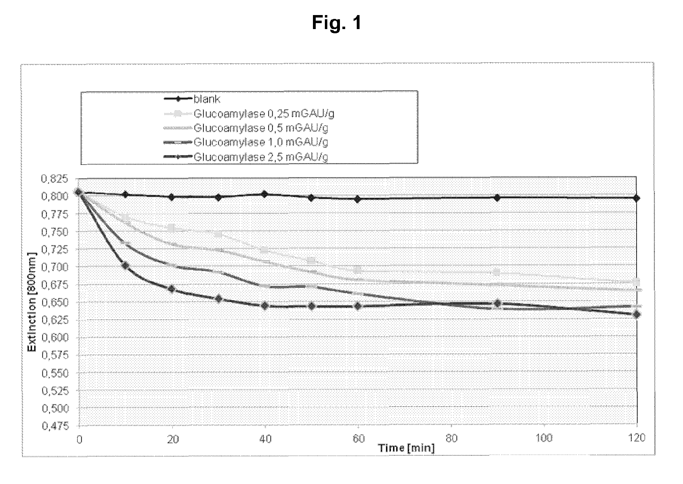

Figure 1 shows yeast lysis with glucoamylase from *Aspergillus niger* alone at different enzyme concentrations. The degree of yeast cell wall lysis is estimated by measuring the extinction at 800 nm. It is shown that glucoamylase from *Aspergillus niger* alone lyses yeast cells, as measured by loss of extinction at 800 nm.

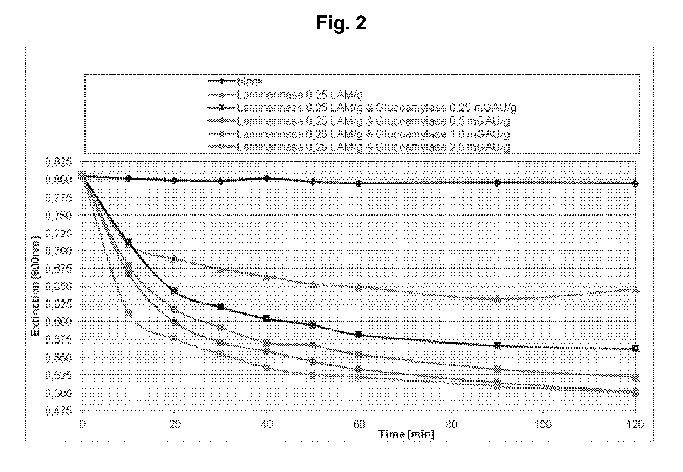

Figure 2 shows yeast lysis with laminarinase (β-1,3-endoglucanase) alone and in combination with glucoamylase (*A. niger*) at different enzyme concentrations. The degree of yeast cell wall lysis is estimated by measuring the extinction at 800 nm. It is shown that the degree of yeast cell wall lysis obtained by a combination of glucoamylase and endoglucanase is more than additive than the degree of yeast cell wall lysis obtained with each of the enzymatic activities alone.

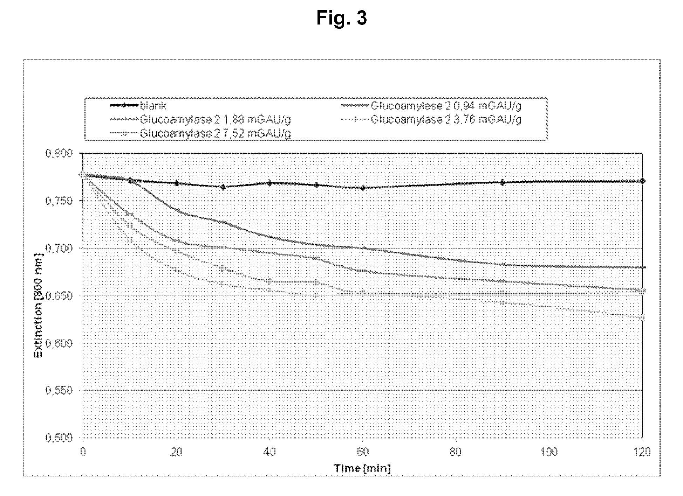

Figure 3 shows yeast lysis with glucoamylase from *Hormoconis resinae* alone at different enzyme concentrations. The degree of yeast cell wall lysis is estimated by measuring the extinction at 800 nm. It is shown that glucoamylase from *Hormoconis resinae* alone lyses yeast cells, as measured by loss of extinction at 800 nm.

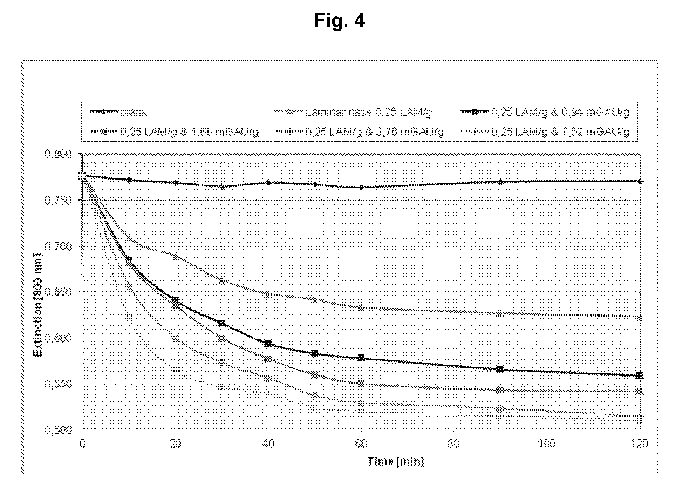

Figure 4 shows yeast lysis with laminarinase (β-1,3-endoglucanase) alone and in combination with glucoamylase (*H. resinae*) at different enzyme concentrations. The degree of yeast cell wall lysis is estimated by measuring the extinction at 800 nm. It is shown that the degree of yeast cell wall lysis obtained by a combination of glucoamylase from *H. resinae* and endoglucanase is more than additive than the degree of yeast cell wall lysis obtained with each of the enzymatic activities alone.

The following examples illustrate the subject-matter of the present invention further.

Referential Example 1

A) Determination of the activity of glucoamylase

1 unit of Glucoamylase activity (GAU/g) (amyloglucosidase) corresponds to said amount of enzyme which catalysis the hydrolysis of 1 $\mu$mol of maltose per minute at 30°C under standard conditions.

The following reagents were used for the determination of the GAU activity:

D(+)-Glucose-monohydrate, Merck, No. 8346, MW 198.17 g mol$^{-1}$
Maltose monohydrate cryst., Merck, No. 5911, MW 360.32 g mol$^{-1}$
Glucose Test Kit, r-biopharm, No. 0716251
Sodium chloride, GR for analysis, Merck, No. 1.06404.5000, MW 58.44 g mol$^{-1}$
Sodium carbonate anhydrous GR, Merck, No. 6392, MW 105.99 g mol$^{-1}$
Acetic acid 100%, Merck No. 1.00063.2511, MW 60,05 g mol$^{-1}$, 1l = 1.05 kg
Sodium acetate anhydrous GR, Merck, No. 6268, MW 82.03 g mol$^{-1}$

**1 M Sodium acetate buffer, pH 4,3**

500 ml of a 1 M sodium acetate solution and 500 ml of an acetic acid solution are prepared in Milli Q water. After that the sodium acetate solution is titrated to pH 4.3 with the acetic acid solution.

The final buffer solution can be stored at 4°C for approximately 6 months.

**Substrate solution, approx. 83 mM**

7.5 g Maltose monohydrate are weighed in a 250 ml volumetric flask and dissolved in Milli Q water. 25 ml 1 M sodium acetate buffer pH 4.3 are added. The volumetric flask is filled up to the mark with Milli Q water. The pH of the solution is controlled and should be pH 4.3.

The substrate solution is prepared daily.

**0.9% Sodium chloride solution**

18 g sodium chloride are weighed in a 2 l volumetric flask and dissolved in Milli Q water. The solution can be stored at 4°C for 3 weeks.

**D- Glucose Test Kit (r-biopharm, No. 0716251)**

The content of bottle 1 is dissolved in 45 ml Milli Q water.

The solution is stable for 4 weeks at 2°-8°C and for 2 months at -15° to-25°C.

The content of bottle 2 is used undiluted.

The solution is stable at 2°-8°C.

**0.05 M Glucose stock solution**

1.9817 g Glucose monohydrate are weighed into a 200 ml volumetric flask and dissolved in 0.9% sodium chloride solution. This solution is prepared daily.

**1% Sodium carbonate solution**

5 g sodium carbonate are weighed into a 500 ml volumetric flask and dissolved in approx. 400 ml Milli Q water. After that Milli Q water is added to make up the volume to 500 ml. The solution can be stored for 3 weeks at 4°C.

**Enzyme solution**

Enzyme samples are dissolved in 0.9% sodium chloride solution.

The method is carried out as follows:

**Main values**

0.5 ml Maltose solution is pipetted into test tubes and equilibrated for 10 min at 30°C in a water bath.

0.5 ml enzyme solution is added. The solution is mixed properly with a test tube mixer and incubated for 30 min at 30°C.

After that 9 ml of the 1% sodium carbonate solution are added. The test tube is closed with a rubber bung and the solution is mixed by twice turning the test tube upside down.

100 $\mu$l of the mixture is pipetted into a new test tube and diluted with 1.9 ml Milli Q water. The solution is mixed with a test tube mixer and after that 1 ml of solution 1 of the D-Glucose test kit (preequilibrated to 20°-25°C) is added. The solution is mixed again and after approx. 3 min at room temperature (approx. 20°-28°C) 20 $\mu$l of solution 2 of the D-Glucose test kit is added. The solution is mixed properly and is stored for about 15 min at room temperature (approx. 20°-28°C).

The solution is mixed again and measured at 365 nm against Milli Q water in a spectrophotometer.

**Blank values**

0.5 ml Maltose solution are pipetted in test tubes and equilibrated for 10 min at 30°C in a water bath.

9 ml 1% sodium carbonate solution are added and the solution is mixed properly.

After that 0.5 ml enzyme solution are added. The solution is mixed again and incubated at 30°C for 30 min. 100 $\mu$l of the solution are pipetted in a new test tube. After this step the following procedure is the same as described for the main values.

B) Determination of the activity of endo-1,3-$\beta$-glucanase (laminarinase)

One endo-1,3-$\beta$-glucanase unit (LAM) is defined as the amount of enzyme producing one nanomol of reducing sugars as glucose in one second (1 ECU=1 nkap).

The following reagents were used:

All solutions are prepared in deionized water, Milli Q or equivalent.

**1. Citrate Buffer (0.05 M, pH 4.8)**

0.05 M solutions of both citric acid ($C_6H_8O_7$*$H_2O$, 10.51 g/l) and sodium citrate ($C_6H_5O_7Na_3$*$2H_2O$, 14.71 g/l) are prepared in water. The pH of the 0.05 M citrate solution is adjusted to 4.8 with the 0.05 M citric acid solution (should require about 667 ml of citric acid solution per 1 l of sodium citrate solution).

**2. Substrate**

1.00 g laminarin (Sigma L9634) is dissolved in citrate buffer and the volume is made up to 100 ml. The powder is dissolved with magnetic stirring for at least one hour, after which it must stand for a further 1 h to clarify.

**3. DNS reagent**

50.0 g of 3,5-dinitrosalicylic acid (Sigma D-0550) are dissolved in about 4 l of water. With continuous magnetic stirring, gradually 80.0 g of NaOH are added and are allowed to dissolve. 1500 g of Rochelle Salt (K-Na-tartrate, Merck 8087) are added in small portions with continuous stirring. The solution may be cautiously warmed to a maximum temperature of 45 °C. It is cooled to room temperature and made up to 5000 ml with water in a volumetric flask. If the solution is not clear, it is filtered through Whatman 1 filter paper.

The assay is carried out as follows:

The sample is diluted in citrate buffer. A suitable dilution will yield an absorbance of 0.20 - 0.25 in the reaction.

1.8 ml of substrate solution are added to each of two test tubes and equilibrated at 50 °C for 5 min. 200 $\mu$l of diluted sample solution are added to one of the tubes and mix with a vortex mixer. After <u>exactly</u> 10 min incubation, 3.0 ml of DNS reagent are added to both tubes and it is mixed. 200 $\mu$l of sample solution are added to that tube (blank) which was incubated without sample. Both tubes are placed in a <u>boiling</u> water bath one at a time. After boiling for <u>exactly</u> 5 min, the tubes are removed and cooled to room temperature. The sample absorbance is measured against the blank at 540 nm. The activity is read from the standard line and the result is multiplied by the dilution factor.

0.1 M stock solution of glucose is prepared. 1.802 g glucose (Merck, 8337) is dissolved in citrate buffer and the volume is made up to 100 ml. The following dilutions are made from the stock solution in citrate buffer:

| Dilution | Glucose concentration $\mu$mol/ml | Activity ECU/ml |
|---|---|---|
| 1:25 | 4.0 | 6.67 |
| 1:15 | 6.67 | 11.11 |
| 1:10 | 10.00 | 16.67 |

Triplicate assays of each standard dilution are performed. 1.8 ml of substrate, 200 $\mu$l of standard dilution and 3.0 ml of DNS reagent are added to a test tube. The mixture is boiled for <u>exactly</u> 5 min, cooled and the absorbances against the reagent blank at 540 nm is measured. The reagent blank is prepared by adding 200 $\mu$l of citrate buffer instead of the standard dilution. To calculate the corresponding endo-1,3-$\beta$-glucanase activity (nkat/ml) the glucose concentration ($\mu$mol/ml) is multiplied by 1000 and divided by the hydrolysis time, 600 s.

C) Determination of the protease activity (UHb$_{4,4}$) (peptidase assay)

One protease unit (UHb$_{4,4}$) is defined as the amount of enzyme activity, which under standard conditions (37°C, pH 4,4,) catalyses the release of trichloroacetic acid soluble haemoglobin compounds equivalent to 1$\mu$mol Tyrosine per minute.

The following reagents were used:

All solutions are prepared in deionized water, Milli Q or equivalent.

**1. Di-sodium hydrogen phosphate solution (0.2 M)**

35.6 g Na$_2$HPO$_4$*2H$_2$O are weighed into a 1l volumetric flask, filled up with "MilliQ" water, stirred until completely dissolved and stored in a refrigerator.

**2. Citric acid 0.1 M**

21 g citric acid monohydrate are weighed into a 1 l volumetric flask, filled up with "MilliQ" water, stirred until completely dissolved and stored in a refrigerator.

**3. Trichloroacetic acid (0.3 M)**

49.02 g trichloroacetic acid DAB6 cryst. are weighed into a 1 l volumetric flask, filled up with "MilliQ" water and dissolved.

**4. Urea, pure (Merck)**

**5. Enzyme solutions**

The enzymes are dissolved in 0.01 M CaCl$_2$-solution. The enzyme solutions should be used within 10 minutes of preparing the final dilution..

The final enzyme dilution should contain 0.5 - 0.75 UHb$_{4,4}$/ml.

**6. Bovine haemoglobin**

MP Biomedicals, LLC (www.mpbio.com)

Bovine Haemoglobin (Cat. No. 100714)

**7. 1 M KH$_2$PO$_4$ - solution**

**Substrate**

Preparation of substrate solution

| Reagent | Amount |
|---|---|
| Haemoglobin | 2.3 g |

(continued)

| Reagent | Amount |
|---|---|
| Milli Q water | 50 ml |
| Urea | 44 g |
| Sodium hydroxide solution 0.5 M | 16 ml |
| 1 M Hydrochloric acid | for pH adjustment |
| $Na_2HPO_4$ 0.2 M | 8.82 ml |
| Citric acid 0.1 M | 11.18 ml |

Add 50 ml Milli Q water to a 300 ml glass beaker and slowly sprinkle in the haemoglobin while stirring. After dissolving the haemoglobin, add the urea. The temperature drops to 0°C and must be rapidly elevated to 25°C by incubation in a water bath (about 60°C). As soon as a temperature of 25°C has been reached, the glass beaker is removed.
Thereafter, the sodium hydroxide solution is added and stirring performed for half an hour at 25°C in order for denaturation of the haemoglobin to take place. Following this the pH is adjusted to pH 4.4 and the 20 ml McIlvaine buffer solution is added. Finally the solution is made up to 140 ml with Milli Q water.

**Method**

**Main values**

5 ml of haemoglobin solution is pipetted into a 50 ml beaker and equilibrated for 10 min at 37°C. Then 1 ml enzyme solution is added while shaking and incubated for precisely 10 min at 37°C.
The reaction is terminated by addition of 10 ml 0.3 m trichloroacetic acid. After being left to stand for about 30 min at room temperature, the reaction suspension is passed through a folded filter (Ederol, grade 14 d = 11 cm).

**Blank values**

5 ml of haemoglobin solution is pipetted into a 50 ml beaker and equilibrated for 10 min at 37°C. Then 10 ml 0.3 m trichloroacetic acid is added, followed by 1 ml enzyme solution, with shaking. The reaction is incubated for precisely 10 min at 37°C.
After being left to stand for 30 min at room temperature, the suspension is treated as described above (main values 3.1).

**Measurement**

The clear filtrates prepared from main and blank values are measured against distilled water at 280 nm in 10 mm quartz cuvettes.
Optimum measurement range: extinction 0.3-0.5 AU.

**Tyrosine calibration curve**

Tyrosine is diluted in Milli Q water at concentrations of between 0 and 1 mmol/l and the extinction determined at 280 nm in 10 mm quartz cuvettes.
The resulting values are plotted on a graph and a straight line fitted to the points to generate a calibration curve. The molar extinction coefficient of tyrosine at pH 4.4 was determined to be 0.11446 l*mmol$^{-1}$ *mm$^{-1}$

**Calculation of protease activity**

The formulae for calculating enzyme activity are as follows:

$$(1) \qquad K_1 = \frac{V}{d * v * t * \varepsilon} \text{ (= constant for experimental parameters)}$$

$$(2) \qquad UHb/g = \frac{\Delta E_{280}}{c} * K_1 * 1000$$

Units

$\varepsilon$ = 0.11446 l*mmol$^{-1}$*mm$^{-1}$
v = 0.001 l (amount of enzyme solution added to reaction)
V = 0.016 l (total reaction volume)
$\Delta$t = 10 min (incubation time)
d = 10 mm (path length of cuvette)
$K_1$ = 1,398 mmol*l$^{-1}$ * min$^{-1}$
$\Delta$E280 = (Difference in extinction at 280 nm between blank and main value)
c = (concentration of enzyme, mmol*l$^{-1}$)

**Example 2 - Enzymatic Yeast Hydrolysis Experiments**

Preparation of yeast

20 g of fresh bakers' yeast is made up to 200 ml with distilled water. Then 8 ml of NaOH solution (50% w/v) is added and the whole is incubated at 85°C for 3 hours.

At the end of the incubation the yeast cells are pelleted using a centrifuge (20 minutes at 10 000g) and the supernatant is discarded. The yeast cells are resuspended in 1000 ml of distilled water, the pH is adjusted to pH 4.0 using citric acid solution (10% (v/v)) and the cells are stored at 4°C for 24 hours.

Before use the yeast cells are diluted to an OD800 of 0.7-0.8 using distilled water and the pH is again adjusted to pH 4.0 using citric acid solution (10% (v/v).

Yeast hydrolysis

30 ml of yeast suspension (OD800 0.7-0.8, pH 4.0) is pipetted into a 50 ml shake flask and pre-incubated at 50°C at 100 rpm for 10 minutes. The OD800 is measured. Then enzyme or water (for the blank) is added to the shake flask and the incubation at 50°C and 100 rpm continues for 120 minutes. At regular intervals a 2 ml sample is taken and the extinction at 800 nm is measured and documented.

The results are presented in figures 1-4. It is to be observed that glucoamylase alone has a lysing effect on yeast cells which effect is synergistically enhanced by combination of a glucoamylase activity with a β-1,3-endoglucanase activity.

**Claims**

1.  Use of a composition comprising at least one polypeptide having glucoamylase activity optionally together with at least one polypeptide having β-1,3-endoglucanase activity for manufacturing wine.

2.  The use of claim 1, wherein the composition further comprises an enzyme selected from peptidases, mannanases, lipases, esterases, beta-1,6-glucanases, alpha-1,3-glucanases alone or in combination.

3.  The use of one of claims 1 or 2, wherein the composition comprises one or more polypeptide(s) having glucoamylase activity, one or more polypeptide(s) having β-1,3-endoglucanase activity and one or more polypeptide(s) having peptidase activity.

4.  The use of one of claims 1 to 3, wherein the polypeptide having glucoamylase activity is selected from fungal glucoamylases, bacterial glucoamylases, yeast glucoamylases, plant glucoamylases, recombinant glucoamylases or an active fragment thereof.

5.  The use of one of claims 1 to 4, wherein the polypeptide having β-1,3-endoglucanase activity is selected from fungal endoglucanases, bacterial endoglucanases, yeast endoglucanases, plant endoglucanases, recombinant endoglucanases or an active fragment thereof.

6. The use of one of claims 1 to 5, wherein the activity ratio of the polypeptide having glucoamylase activity and the polypeptide having β-1,3-endoglucanase activity is between 1 GAU : 100.000 LAM and 100 GAU : 1 LAM.

7. A process for manufacturing wine which comprises a process of lysing yeast cell walls which comprises exposing said yeast cell walls to a composition comprising at least one polypeptide having glucoamylase activity and optionally at least one polypeptide having β-1,3-endoglucanase activity.

8. The process of claim 7, wherein the composition further comprises an enzyme selected from peptidases, mannanases, lipases, esterases, beta-1,6-glucanases, alpha-1,3-glucanases alone or in combination.

9. The process of one of claims 7 or 8, wherein the composition comprises one or more polypeptide(s) having glucoamylase activity, one or more polypeptide(s) having β-1,3-endoglucanase activity and one or more polypeptide(s) having peptidase activity.

10. The process of one of claims 7 to 9, wherein the polypeptide having glucoamylase activity is selected from fungal glucoamylases, bacterial glucoamylases, yeast glucoamylases, plant glucoamylases recombinant glucoamylases or an active fragment thereof.

11. The process of one of claims 7 to 10, wherein the polypeptide having β-1,3-endoglucanase activity is selected from fungal endoglucanases, bacterial endoglucanases, yeast endoglucanases, plant glucoamylases, recombinant endoglucanases or an active fragment thereof.

12. The process of one of claims 7 to 11, wherein the activity ratio of the polypeptide having glucoamylase activity and the polypeptide having endoglucanase activity is between 1 GAU : 100.000 LAM and 100 GAU : 1 LAM.

# Fig. 1

## Fig. 2

## Fig. 3

## Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9623579 A
- WO 2008037777 A
- WO 2008110632 A
- WO 2007042577 A
- WO 92100381 A
- WO 0004136 A
- WO 08402921 A
- US 7413879 B
- US 5665585 A
- WO 9928448 A
- US RE32153 E
- US 4587215 A
- EP 0135138 A
- WO 8601831 A
- US 6284509 B
- US 4218418 A, Chao

### Non-patent literature cited in the description

- **SALAZAR ; ASENJO.** Enzymatic lysis of Microbial cells. *Biotech. Lett.,* 2007, vol. 29, 985-994
- **LIPKE ; OVALLE.** *Cell Wall Architecture in Yeast: New Structure and New Challenges,* 1998
- **CABIB et al.** Carbohydrates as structural constituents of yeast cell wall and septum. *Pure & Appl. Chem.,* 1991, vol. 63, 483-489
- **MANNERS et al.** The Structure of a ß-1,3-glucan from yeast cell walls. *Biochem. J.,* 1973, vol. 135, 19-30
- **BOEL et al.** *EMBO J.,* 1984, vol. 3 (5), 1097-1102
- *Agric. Biol. Chem.,* 1991, vol. 55 (4), 941-949
- **CHEN et al.** *Prot. Engng,* 1996, vol. 9, 499-505
- **CHEN et al.** *Prot. Engng,* 1995, vol. 8, 575-582
- **CHEN et al.** *Biochem. J,* 1994, vol. 301, 275-281
- **FIEROBE et al.** *Biochemistry,* 1996, vol. 35, 8698-8704
- **LI et al.** *Protein Engng.,* 1997, vol. 10, 1199-1204
- E.C.3.2.1.39 and E.C. 3.2.1.6, Enzyme Nomenclature. Academic Press, Inc, 1992
- **NOBE et al.** *Biosci. Biotechnol. Biochem.,* 2004, vol. 68, 2111-2119
- **MURRAY et al.** *Enz. Microb. Technol.,* 2001, vol. 29, 90-98
- **LLOBERAS et al.** *Europ. J. Biochem.,* 1991, vol. 197, 347-353
- **HOFEMEISTER et al.** *Gene,* 1986, vol. 49, 177-187
- **HOFEMEISTER et al.** *J. Basic Microbiol,* 1988, vol. 28, 1-10
- **CANTWELL et al.** *Gene,* 1983, vol. 23, 211-219
- **ZVERLOV et al.** *Microbiol.,* 1997, vol. 143, 1701-1708
- **ADAMITSCH et al.** *Lett. App. Microbiol.,* 2003, vol. 36, 227-229
- **CONWAY et al.** *Can. J. Microbiol.,* 2001, vol. 47, 18-24
- **SHIMOI et al.** *J. Biol Chem,* 1992, vol. 267, 25189-25195